(19) Europäisches Patentamt
European Patent Office
Office européen des brevets

(11) **EP 2 243 419 B1**

(12) **EUROPÄISCHE PATENTSCHRIFT**

(45) Veröffentlichungstag und Bekanntmachung des Hinweises auf die Patenterteilung:
**15.08.2012 Patentblatt 2012/33**

(51) Int Cl.:
**A61B 3/028** (2006.01)    **A61B 3/02** (2006.01)

(21) Anmeldenummer: **09005757.1**

(22) Anmeldetag: **24.04.2009**

(54) **Integrale und differentiale visuelle Objekte zur Erhebung und Optimierung des Sehvermögens**

Integral and differential visual objects for examination and optimization of vision

Objets visuels intégraler et différentiels pour l'examen et l'optimisation de l'acuité visuelle

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(43) Veröffentlichungstag der Anmeldung:
**27.10.2010 Patentblatt 2010/43**

(73) Patentinhaber: **Stuetz, Ignaz Alois**
**4212 Neumarkt im Muehlkreis (AT)**

(72) Erfinder: **Stuetz, Ignaz Alois**
**4212 Neumarkt im Muehlkreis (AT)**

(74) Vertreter: **Piermayr, Alexander**
**Stelzhamerstraße 12**
**4020 Linz (AT)**

(56) Entgegenhaltungen:
**WO-A1-92/10970    WO-A1-02/076301**
**GB-A- 2 247 087    GB-A- 2 264 366**
**US-A- 4 412 729    US-A1- 2006 152 675**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

**1. Das technische Gebiet**

In der Optometrie, der Ophthalmologie sowie bei Screennigs werden Messverfahren eingesetzt, bei denen visuelle Objekte Verwendung finden. Außer objektiven Verfahren werden, um die Reaktion der Testperson zu beobachten, Dinge, helle oder dunkle Punkte, regelmäßige Muster und Sehzeichen verwendet. Zur Bestimmung des Sehvermögens werden den Testpersonen periodische Muster oder standardisierte Darstellungen präsentiert, wobei außer dem genormten Landoldtring diesem angeschlossene Optotypen, Figuren, Kinderbilder etc. auf einer Testfläche (Tafel, Bildschirm, Projektion) dargestellt werden, die nach gängiger Definition heller sein soll als die Umgebung.

**2. Stand der Technik**

Zur Optimierung werden objektive und subjektive Verfahren angewandt, zur Analyse verwenden Professionisten verschiedenste Messverfahren des Sehvermögens. Bevorzugt erhoben wird die zentrale Sehschärfe durch Verwendung von Landoldtringen oder äquivalenten Buchstaben und Ziffern, sogenannten Optotypen, die vom Probanden vorzulesen sind. Analphabeten und Kindern werden Snellenhaken, Kinderbilder oder figurale Darstellungen (z.B. nach Lea Hyvärinen) offeriert, die zu zeigen oder zu benennen sind, wie im nächstliegenden Stand der Technik beschrieben, der durch die Dokumente GB 2247087 und US 2006/0152675 gegeben ist.

Zur Bestimmung der zentralen Sehschärfe (bevorzugt aber zur Messung der Kontrastleistung) werden auch rechteckige bis sinusförmige Gittermuster oder periodische Mehrfachringe verwendet. Anders als bei den oben beschriebenen Testfeldern für Schzcichen tritt hier die Leuchtdichte des Testfeldes gegenüber dem Umfeld nicht besonders hervor. Entweder muss die Testperson die Richtung der Streifen bzw. bei bewegter Darstellung die Art oder Richtung der Bewegung angeben, oder es genügt das Erkennen überhaupt, weil die durchschnittliche Leuchtdichte der helleren und dunkleren Anteile gleich ist der umgebenden Helligkeit, sodass bei zu geringem Sehvermögen die Grenzlinie der (meistens kreisrunden) Fläche, auf der die periodische Darstellung erfolgt, sich nicht mehr von der neutralen Umgebung abhebt.

In den letzten Jahren wird der Erhebung des Kontrastsehvermögens stärkere Aufmerksamkeit gewidmet. Dazu wird bei periodischen Darstellungen die Helligkeit der dunkleren und helleren Elemente einander angenähert, bis sie von der Testperson nicht mehr unterschieden werden können (z.B. Vistech-Tafel). Im Grohinger-Edge-Test werden zur Kontrastmessung Kreise in zwei Hälften geteilt, die sich bei hohem Kontrast beginnend im Laufe der Testreihe immer weniger in der Leuchtdichte unterscheiden. Zur Prüfung werden die Kanten in verschiedene Richtungen orientiert.

Auch die schon für die Visusprüfung verwendeten schwarzen Zeichen werden zur Kontrastmessung verwendet, indem sie in Richtung "weiß", also heller Hintergrund, verändert werden. Die Präseritationsweisen der kontrastarmen Sehzeichen unterscheiden sich. Bei den einen muss hei einem festgelegten Kontrast das kleinste Sehzeichen benannt werden (LCS-Test n. Buser, Bailey-Chart). Bei anderen Testverfahren bleibt die Größe gleich, während der Kontrast verändert wird, indem die Leuchtdichte der Optotypen dem weißen Hintergrund angenähert wird (Pelli-Robson-Tafel, Lea Hyvärinen Test).

Die Perimetrie wird in der Praxis vor allem für Screening und diagnostische Zwecke verwendet. Dabei wird die Sensibilität bzw. Kontrastempfindlichkeit auf der "ganzen" Netzhaut gemessen. Ziel ist, den genauen Ort, die Ausdehnung und die Tiefe der pathologischen Veränderung herauszuarbeiten, Unkritisch werden die dabei ermittelten Werte extrapoliert und als "peripheres Sehvermögen" interpretiert. Es gibt Handperimeter (nach Goldmann) und die moderne Computerperimetrie, die Kampimetrie (auf einem ebenen Schirm) und entsprechende elektronische Darstellungen auf (Flach-)Bildschirmen. Bevorzugt werden helle Punkte vor neutralgrauem Hintergrund dargestellt, neuerdings auch dunkle Punkte, die nicht z.B. durch Medientrübungen in das Umfeld überstrahlen können. Weil Erkrankungen (der Netzhaut) des Auges vor allem bestimmte Farbkontraste reduzieren, werden auch farbige Punkte sowie Farbpunkte mit umlaufendem Farbring in Komplementärfarben dargestellt. In jüngerer Zeit werden, in Anlehnung an die Messung der zentralen Kontrastleistung, auch rund um das Fixationszentrum sowohl Gittermuster, in linear- wie in kreisförmig-periodischer Anordnung, dargestellt, als auch verschieden große Optotypen mit geringem Kontrast präsentiert.

Für die mobile Sicherheit des Menschen ist die Wahrnehmung von Bewegungen im Gesichtsfeld wichtig. Daher versucht man, den Winkelabstand (in Grad) vom Fixationszentrum in den verschiedenen Richtungen zu bestimmen, ab welchem eine Bewegung im Gesichtsfeld wahrgenommen wird, bzw. ob an einem bestimmten Netzhautort eine Bewegung wahrgenommen wird. Zum Ersten werden Objekte im Gesichtsfeld bewegt und zum Zweiten kleine, sich bewegende Optotypen (z.B. rotierende Landoldtringe) ebenso wie oszillierende Gittermuster außerhalb des Fixationszentrums präsentiert.

Zur Bestimmung der Lesefähigkeit in Bezug auf die Textgröße werden Lesetafeln verwendet, die Texte in verschiedenen Größen im Hochkontrast darstellen. Um Kontrastschwächen beim Lesen zu ermitteln wurden Tafeln mit kontrastarmen ("hellgrauen") Texten entwickelt - sie werden aber in der Praxis kaum eingesetzt. Zur Beurteilung der qualitativen Lesefähigkeit gibt es standardisierte Lesetests, bei denen außer der Lesegeschwindigkeit das Textverständnis etc. wesentliche Bewertungskriterien darstellen.

Zur Quantifizierung einer Blendung sind Geräte zur Beurteilung der Fahrtüchtigkeit beim Lenken eines KFZ entwickelt worden, deren Ergebnisse auch nur gerätespezifisch evaluiert werden (können) - es wird bei Leuchtdichten, die dem Dämmerungssehen entsprechen, die zentrale Sehschärfe und die Kontrastleistung ermittelt, auch unter Einwirkung von blendendem Seitenlicht. Die alltagsrelevante Problematik der reduzierten Kontrastleistung von Testpersonen, zentral und um das Fixationszentrum herum, sowie Adaptionsprobleme (absolut und in der Geschwindigkeit), können für die Lichtsituationen, wie sie dem Lenker eines KFZ im Alltag begegnen, mit den bekannten Adaptometern, anders als mit der vorliegenden Erfindung, nicht reproduziert werden.

Die vorliegende Erfindung bezieht sich nicht auf objektive Verfahren zur Brillenglasbestimmung. Dagegen können die visuellen Objekte durchaus für subjektive Verfahren zur Optimierung des Sehvermögens durch Korrektion verwendet werden.

### 3. Beschreibung der Erfindung im Detail

Bei der vorliegenden Erfindung handelt es sich um nicht-periodische visuelle Objekte zur Erhebung und Optimierung des Sehvermögens. Der Kernpunkt der Erfindung liegt darin, dass die angesprochenen singulären Objekte selbst durchschnittlich genauso hell sind wie das Testfeld, also auf kleinem Raum Leuchtdichtewechsel darstellen, die einander aufheben. Mathematisch entsprechen die "integralen Objekte" der zweiten Ableitung einer (visuellen Objekt-)Kante, die "differentialen Objekte" der dritten Ableitung; das Integral ihrer Leuchtdichte L ist bei beiden gleich der Leuchtdichte des Umfeldes.

Die Darstellung geschieht durch technisch gängige Verfahren: entsprechend gestaltete Gegenstände, von vorne beleuchtete bzw. von hinten durchleuchtete Tafeln, Projektion auf eine Tafel, durch Bildschirme verschiedenster Technik sowie durch Projektion direkt auf die Netzhaut, ob durch Refraktion oder Interferenz.

Zur Erhebung des zentralen Sehvermögens können verschiedene Parameter bestimmt werden wie z.B.: Sehschärfe, Kontrastleistung und -schwelle, Stereosehen, Adaptionsfähigkeit und - geschwindigkeit, Blendempfindlichkeit, etc.; dies alles bei verschiedenen Helligkeiten, Farben und Farbtemperaturen. Die vorliegende Erfindung erlaubt die raschere und präzisere Bestimmung dieser bekannten und noch anderer Parameter im Fixationszentrum, aber auch im Gesichtsfeld darum herum (z.B. Vergrößerungsbedarf zum Lesen ) sowie in der Peripherie (funktionales Gesichtsfeld). Es lassen sich zumutbare Belastungsdauer und -fähigkeit reproduzierbar erheben, indem bei entsprechender Testanordnung Veränderungen der Grenzwerte sowie der Reaktionszeit erfasst werden. Durch die Variabilität der Objektdarstellung ("beliebige" Objektgröße, Kontraste hoch bis niedrig, Beleuchtungen für photopisches bis skotopisches Sehen , statische, dynamische oder oszillierende Präsentation , fest oder mobil für zentrale oder periphere Netzhautorte, etc.) werden einige, heute noch zu wenig beachtete, aber für den Alltag sehr wichtige Sehleistungen mess- und vergleichbar, wie etwa Kontrastleistung und (Re-)Adaptionsgeschwindigkeit bei alltäglichen Licht- und Blendungssituationen oder die Größe des spontan verfügbaren Gesichtsfeldes für niedrige Kontraste.

Ebenso sind die vorliegenden Objekte für die Optimierung des Sehvermögens durch subjektive Brillenglasbestimmung nach gängigen Verfahren einsetzbar und stellen bei Abschluss desselben einen Nachweis für die qualitativ hochwertige Durchführung dar. Nicht zuletzt eignen sie sich hervorragend für Screening-Verfahren, bei denen mit geringem Aufwand nach relativ kurzer Zeit die signifikanten Werte vorliegen sollen.

Zum Unterschied von bisher gängigen "gefüllten" Zeichen oder Strichfiguren als visuelle Objekte, unterscheidet sich zunächst die Leuchtdichte des Testfeldes, auf dem die Objekte dargestellt werden, nicht wesentlich von der umgebenden Raumhelligkeit. Ein gewisser Unterschied wird in der praktischen Anwendung nicht vermeidbar sein, muss aber so niedrig gehalten werden, dass die Differenz selbst bei hochgradig adaptionsschwachen und blendempfindlichen Menschen keinen messbaren Einfluss auf das Ergebnis hat.

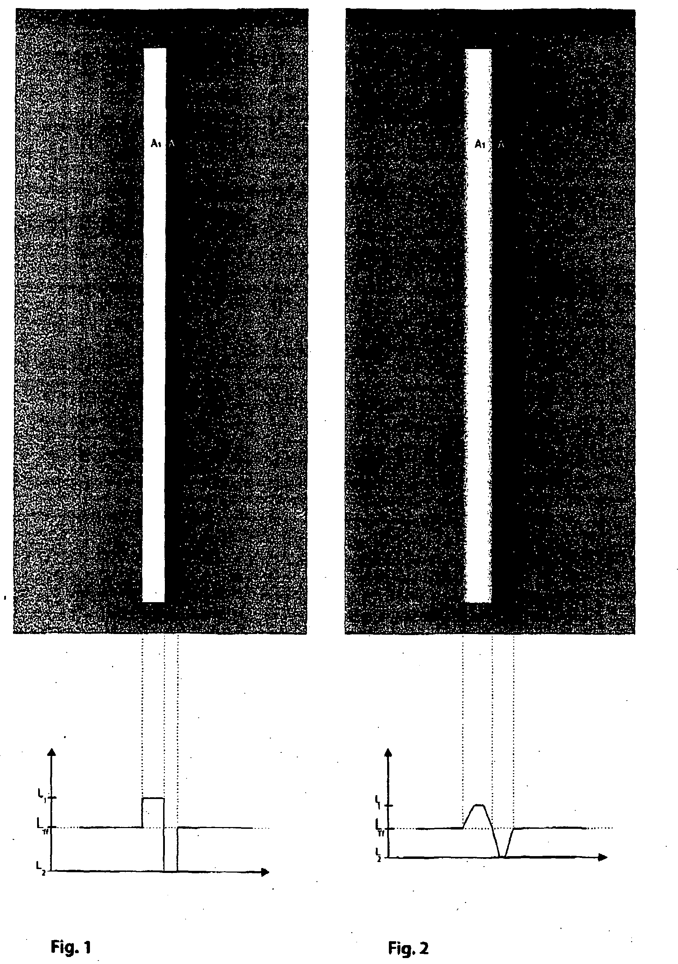

Als jedoch zentrales Merkmal der Erfindung unterscheiden sich auch die Objekte selbst in ihrer durchschnittlichen Leuchtdichte nicht von der Leuchtdichte des Testfeldes. Dies ist in Fig. 1 dargestellt: der Mittelwert der Leuchtdichte L des Objektes wird durch einen Quotienten gebildet: im Zähler scheint die Summe der Lichtstärken aus Leuchtdichte $L_1$ mal $A_1$ addiert mit $L_2$ mal $A_2$ auf, im Nenner die Gesamtfläche $A_1 + A_2$, wobei dieser durchschnittliche Wert nun der Leuchtdichte des Testfeldes $L_{Tf}$ entsprechen soll. In der Praxis wird dies nicht immer exakt realisierbar sein. Jedenfalls soll gelten, dass der Leuchtdichteunterschied zwischen den reziproken Flächen ($A_1$, $A_2$) des Objektes höher ist als jede Differenz der Leuchtdichte einer einzelnen Fläche des Objektes zu der des Testfeldes:

$$|L_1 - L_2| > |L_1 - L_{Tf}| \quad und \quad |L_1 - L_2| > |L_2 - L_{Tf}|$$

Die Übergänge an den Grenzen zwischen den Flächen, sowohl im Objekt selbst als auch zwischen Objekt und Testfeld, können unterschiedlich ausgeführt sein. Außer der abrupten, scharfen Änderung der Leuchtdichte an einer Grenzlinie kann diese auch allmählich erfolgen. Diese Übergänge sind mit gängigen mathematischen Formeln beschreibbar, durch einfache lineare Funktionen verschiedener Steigung (vgl. Fig. 2) ebenso wie durch komplexe Funktionen (über eine Fourier-Transformation).

Um die Grenzwerte des Sehvermögens möglichst in alltäglichen Lichtsituationen zu erheben, ist es erforderlich, diese bei der konkreten Testanordnung zu reproduzieren. Dafür sind Präsentationen herzustellen, bei denen Raumleuchtdichte und Testfeldleuchtdichte in ihrer Helligkeit beispielsweise sonnendurchflutetem Tageslicht entsprechen, beschattete Lichtsituationen im Freien wie in Räumen (photopisches Sehen) wiedergeben, dem Dämmerungssehen (mesopisches Sehen) oder auch dem Nachtsehen (skotopisches Sehen) entsprechende Leuchtdichten reproduzieren. Um z.B. die Einfahrt in einen Tunnel mit einem KFZ bei Tag zu simulieren muss die Leuchtdichte entsprechend rasch abgesenkt werden, zur Darstellung der Wirkung von Gegenverkehr bei Dunkelheit ist eine (vorübergehende) Blendungssituation herzustellen, nach der dann beispielsweise die Readaptionsgeschwindigkeit erhoben wird.

Visuelle Wahrnehmung besteht grundsätzlich darin, dass der Rand eines Dinges, die Objektkante erfasst wird (mit "Rand" oder "Objektkante" ist die Grenze zwischen dem Ding und der Umgebung bzw. dem Hintergrund gemeint). Alle Orte, an denen sich etwas gleich oder ähnlich ändert, gehören zusammen, sodass Lebewesen mit einem Sehsinn sehr individuell Gestalten und Formen bilden oder Einschätzungen in Rückkoppelung mit der Erinnerung für Bilder oder Bewegungsmuster vornehmen, sodass sie sehr rasch darauf reagieren können.

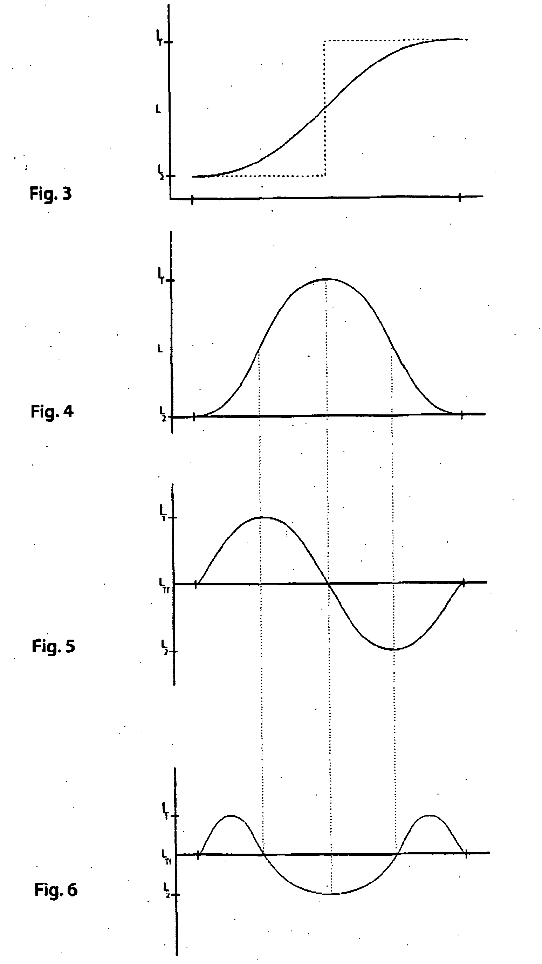

Diese Änderung kann sich auf unterschiedliche Helligkeit oder Farbe, die Struktur, Bewegungsmuster, auf Schattenbildung usw. beziehen. Der einfachste Fall der ausschließlich statischen Leuchtdichtenänderung ist schematisch in Fig. 3 als punktierte Linie dargestellt. Auch bei bester Einstellung wird im Auge jede Objektkante "verschwommen" auf die Netzhaut abgebildet. Fachlich wird dies als "optische Übertragungsfunktion (OTF)" bezeichnet, nährungsweise gezeichnet als durchgezogene Linie in Fig. 3. In erster Annäherung wird der Grad der Leuchtdichteänderung erhoben, somit die Steigung der Funktion. Mathematisch gesprochen wird die erste Ableitung gebildet (Fig. 4). Das entspricht in der Realität einer Linie und der Objektdarstellung durch Zeichnung. Diese Form der Abstraktion ist bereits bei Höhlenzeichnungen anzutreffen.

Betrachtet man die Steigung dieser ersten Ableitung, wird mathematisch die zweite Ableitung gebildet (Fig 5). Diese beschreibt nun nur mehr den Charakter der Änderung, der Graph bewegt sich um die x-Achse herum. Bildet man das Integral dieser Funktion über den Bereich der gesamten Abweichung, so ist diese gleich Null (bzw. gleich der Leuchtdichte L): es entsteht eine "fotometrische Symmetrie" (eine Art "Leuchtdichtengleichgewicht"), indem die Flächen zwischen Graph und x-Achse ober- und unterhalb dieser einander aufheben. Die "integralen Objekte" sind im Sinne der Erfindung Darstellungen (als geometrische bzw. reelle interpretation) der zweiten Ableitung einer Objektkante, wobei in der Realität die x-Achse der durchschnittlichen Leuchtdichte eines integralen Objektes entspricht - sie ist gleich der Leuchtdichte des Testfeldes (prinzipiell in Fig. 1 und Fig. 2 dargestellt).

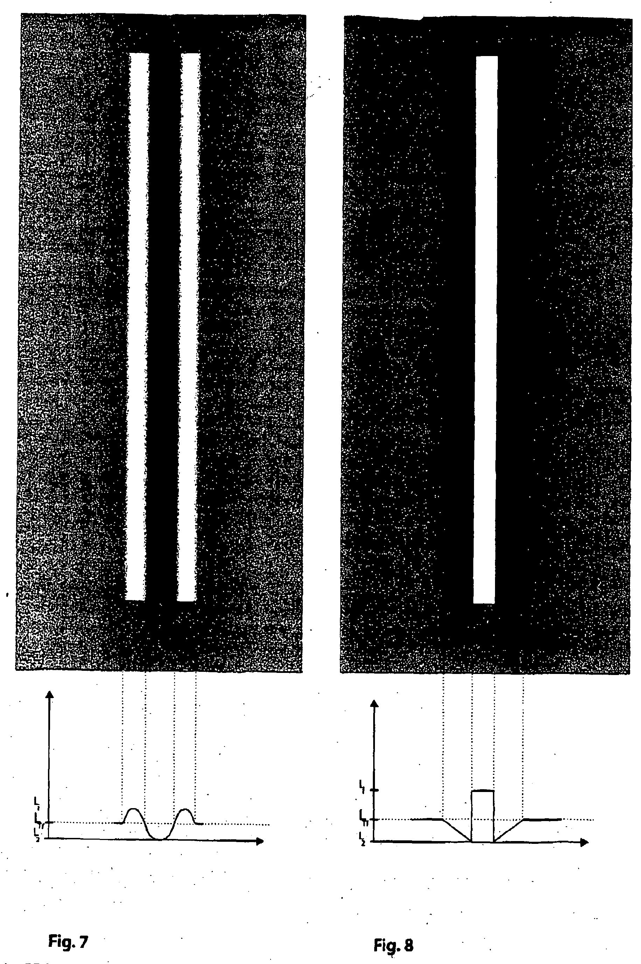

Um die differentialen Objekte herzuleiten wird die dritte Ableitung dieser Funktion gebildet (Fig. 6). Wie oben ist auch hier das Integral der Funktion über den gesamten Bereich der Abweichung gleich Null: die beiden Flächen (zwischen Graph und x-Achse) oberhalb sind in Summe gleich derjenigen unterhalb der x-Achse. Nun umschließen, horizontal betrachtet, zwei äußere Flächen mit gleicher Leuchtdichte eine innere Fläche mit gegengleicher Leuchtdichte, es ergibt sich eine Art Polarität. Die "differentialen Objekte" sind im Sinne der Erfindung Darstellungen der dritten Ableitung einer Objektkante (Fig. 7.). Die x-Achse entspricht in der reellen Interpretation der durchschnittliche Leuchtdichte eines differentialen Objektes, die Distanz der beiden Nulldurchgänge ergibt den Abstand der beiden Gegenpole, sodass zum variablen Kontrast unabhängig davon auch ein beliebiger Auflösungswinkel für Beobachter hergestellt werden kann.

Der Verlauf der Leuchtdichteänderung ist umkehrbar; man kann auch von positiver und negativer Darstellung sprechen (Fig. 8). Je nach Aufgabenstellung bei Sehtests bzw. der Fragestellung bei der Brillenglasbestimmung wird sich in der Praxis und durch Reihentests zeigen, mit welcher Darstellungsart diesen am besten entsprochen werden kann.

Da integrale und differentiale Objekte aus einem neutralen Umfeld hervortreten, kann unter Verwendung geeigneter Techniken die Dynamik der Darstellung variiert werden. Einerseits kann sie abrupt sein - das Objekt ist plötzlich da. Andererseits kommt ein allmähliches Erscheinen in Betracht, im Sinne einer dynamischen Steigerung des Kontrastes, bei differentiale Objekten auch in Form einer Änderung des Sehwinkels. Das gilt auch umgekehrt, dass also der Kontrast dieser Objekte, bei differentialen Objekten auch das Abstandskriterium, verkleinert werden bis die Darstellungen für den Beobachter entschwinden.

Die positive und negative Darstellung kann auch mit der Dynamik der Darstellung kombiniert werden. Diese Oszillationen der Präsentation können abrupt erfolgen oder (z.B. sinusförmig) schwingend, über einen gewissen Zeitraum geichbleibend sein oder sich in Intensität oder Frequenz ändern.

Abgesehen von den oben beschriebenen Darstellungsarten können integrale und differentiale Objekte in Art und Form sehr unterschiedlich (große) Figuren bilden, von denen einige beispielhaft angeführt werden. Eine abschließende Auflistung ist auf Grund der vielfältigen Darstellungsmöglichkeiten und ihrer Kombinierbarkeit naturgemäß nicht möglich. Ihnen allen ist jedoch gemein, dass sie nicht-periodische, symmetrische bzw. polare Änderungen der Leuchtdichte auf oder vor dem Testfeld als Helligkeitsbasis sind, also in ihrer durchschnittlichen Leuchtdichte der des Testfeldes entsprechen (Integral = $L_{Tf}$),

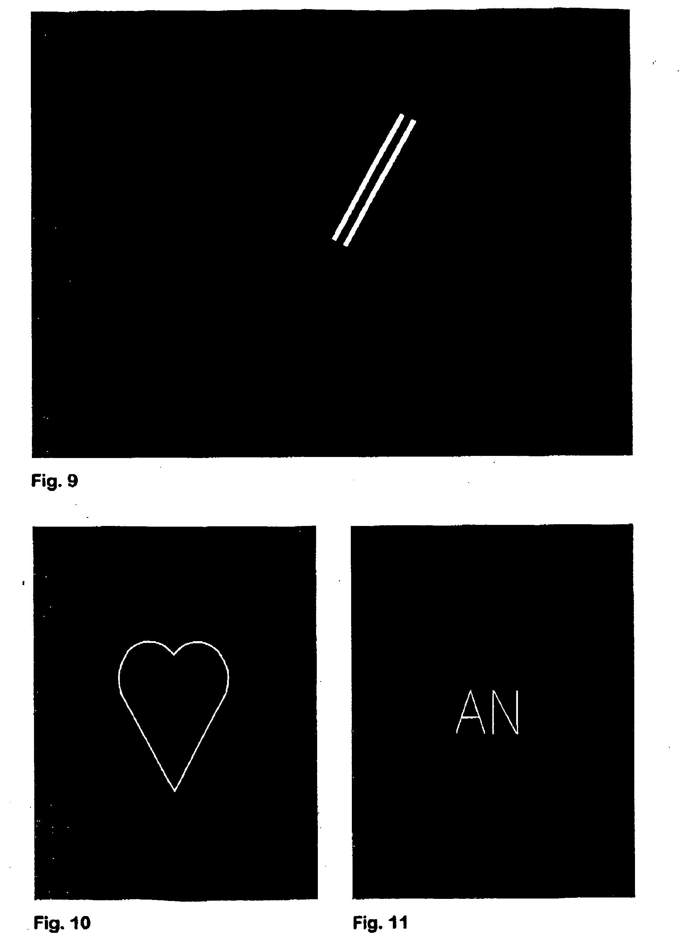

Ein einfaches Objekt ist eine entsprechend dieser Vorschrift hergestellte gerade Linie, wie in Fig. 9 als differentiale Linie. Wenn diese z.B. wie ein Uhrzeiger in ein Ziffernblatt eingefügt wird, besteht die Aufgabe für die Testperson, die Uhrzeit anzugeben. Damit können, z.B. bei unkorrigiertem Astigmatismus, auch je nach Zeigerrichtung unterschiedliche Sehleistungen herausgearbeitet werden. Mit differentialen oder integralen Linien können Buchstaben, Ziffern, Wörter, einfache geometrische Zeichen, Piktogramme etc. gebildet werden (Fig. 10 und 11 mit integraler Linie). Für alle mittels integraler Linie dargestellten Objekte gilt, dass Kontrast, Kantengestaltung, Linienbreite etc. bei unveränderter Gesamtgröße des Objektes beliebig variierbar sind. Bei Verwendung einer differentialen Linie ist zusätzlich ein Abstand als Kriterium für das minimum separabile (entspricht der Sehschärfe) herstellbar, der in großem Bereich bei konstanter Objektgröße veränderbar ist!

Demgegenüber kann im Sinne der Erfindung ein sehr kleines Objekt hergestellt werden, beispielhaft bezeichnet als "Differential-Ring": ein heller Kreis wird von einem dunklen Ring umschlossen oder umgekehrt ein dunkler Punkt von einem helleren Ring - die Form muss nicht kreisförmig sein, es erscheint aber aus heutiger Sicht am sinnvollsten. Ebenso kann aus einer integralen Linie ein Ring geformt werden, der im Sinne der Durchgängigkeit der Bezeichnung als "Integralring" zu benennen wäre.

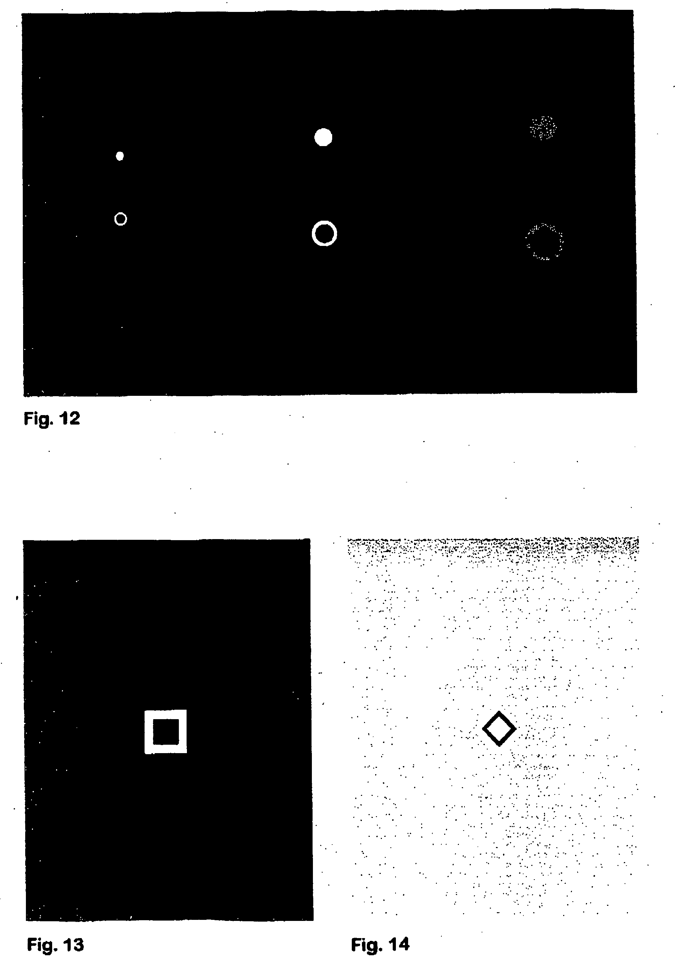

Es sind die verschiedensten integralen und differentialen Objekte gestaltbar - sie können und brauchen hier nicht erschöpfend aufgezählt werden. Eine Gruppe sei explizit erwähnt, weil es dabei zum Unterschied vom Differential-Ring auch wirklich Sinn macht, verschiedene Formen zu unterscheiden, wie es für bestimmte Fragestellungen bei Sehtests benötigt wird (Fig. 13 und 14.) Sie sind etwas größer als der Differential-Ring und unterscheiden sich von diesem dadurch, dass im Zentrum eine weitere Fläche mit der Leuchtdichte der äußersten Fläche vorhanden ist. Diese differentialen Objekte haben also eine Innen-, eine Zwischen- und Außenfläche. Dabei können die Begrenzungen der Flächen ähnliche Formen aufweisen oder - wie in Fig. 14 - völlig unterschiedlich hergestellt werden, etwa auch als Buchstaben in einer beliebig geformten Fläche. Natürlich gilt auch für diese Objekte das zentrale Merkmal der Erfindung, dass die durchschnittliche Leuchtdichte des gesamten Objektes jener des Testfeldes entspricht.

Integrale und differentiale Objekte können nicht nur, wie bereits oben beschrieben, variabel dargestellt werden; sie können, auch in sich, in Größe oder Form dynamisch verändert werden oder oszillieren. Dabei kann eine geometrische Form in eine andere wechseln, hinter- oder nebeneinander präsentierte Wörter einen Text bilden oder z.B. ein Strichmännchen sich wie in einem Video bewegen. Weiters sind auf einer Testfläche mehrere integrale und differentiale Objekte darstellbar, die unabhängige voneinander erscheinen und verschwinden, ihren Ort oder die relative Position zueinander verändern, miteinander eine sinnvolle Gestalt bilden, die sich später wieder auflöst, etc.

Bei getrennter Projektion in die Augen oder durch Verwendung gängiger Trennverfahren bei Präsentationen können gleichzeitig ins rechte und linke Auge ausschließlich oder zusätzlich unterschiedliche Objekte auf die Netzhaut abgebildet werden, oder es werden mit gleicher Technik eines (oder mehrere) von mehreren visuellen Objekten ins rechte und linke Auge zwar gleichartig, aber mit gering horizontal verschobener Position, dem sogenannten differenzierten Stereowinkel, abgebildet. Damit sind integrale und differentiale Objekte bevorzugt zur Prüfung und Optimierung des Binokularsehens sowie des räumlichen Tiefensehens (Stereopsis) einzusetzen. Die stereoskopische Präsentation von integralen und differentialen visuellen Objekten ist mit allen oben genannten Variationen, dynamischen Darstellungsarten sowie (ineinanderlaufenden) Mehrfachdarstellungen kombinierbar.

Bisher hat sich die Beschreibung der Erfindung auf Darstellungen der Objekte in (einheitlichem) schwarz-grau-weiß beschränkt. Außer den oben genannten vielfältigen Variationen können die Flächen der integralen und differentialen Objekte samt Testfläche auch beliebig mit verschiedenen Lichtarten, Farbbändern und Spektralfarben gefüllt werden, wobei die Prinzipien der Symmetrie und der Polarität erhalten bleiben, sodass bevorzugt unterschiedliche Leuchtdichten bei sonst gleichen Spektralfarben, -anteilen, -bändern oder Farben innerhalb eines integralen oder differentialen Objektes zum Einsatz kommen. Z.B. kann auch, um die chromatische Adaption der Beobachter (sogenannte "Farbumstimmung") zu erschweren, das Testfeld neutralgrau sein, während die Objekte darauf in verschiedenen Farben gleichzeitig nebeneinander präsentiert werden.

Um eine alltägliche Blendungssituation reproduzieren zu können muss vom bisher geltenden Prinzip, dass die Leuchtdichte des Umfeldes möglichst gleich ist der des Testfeldes, (für diesen Zweck) abgegangen werden. (Beibehalten wird definitionsgemäß, dass die durchschnittliche Leuchtdichte der Objekte der des unmittelbaren Umfeldes im Testfeld entspricht.) Zur Reproduktion einer peripheren Blendung (= Blendung durch eine hellere Fläche im Gesichtsfeld, typisch: tiefstehende Sonne) wird in Intensität und Größe alltägliche Lichtsituationen nachbildend, eine höhere Leuchtdichte im Gesichtsfeld erzeugt und während dieses Zeitraums bzw. danach das Sehvermögen mittels integraler und differentialer Objekte ermittelt. Umgekehrt ist zur Herstellung einer zentralen Blendung (= Blendung durch das Testfeld selbst, typisch: Zeitunglesen bei direkter Sonneneinstrahlung) die Testfläche mit entsprechend größerer Helligkeit als das übrige Gesichtsfeld auszustatten.

Bei den meisten bisher gängigen Schtests mittels Optotypen ist deren Vorhandensein unzweifelhaft (minimum visibile ist positiv). Die Aufgabe besteht darin, die Details zu differenzieren (minimum separabile ist fraglich). Zum Unterschied davon fällt bei integralen und differentialen Objekten die Erkennbarkeit mit der Unterscheidbarkeit (mit wenigen Ausnahmen, die sich aufgrund der Art der Fragestellung ergeben) zusammen. Damit ist die Erhebung des Sehvermögens unter Anwendung integraler und differentialer Objekte bei entsprechender Präsentationstechnik grundsätzlich "non-frustrativ". Reicht nämlich das Auflösungsvermögen, die Kontrastleistung etc. nicht aus, so sind die integralen und differentialen visuellen Objekte auf Grund der definitionsgemäßen Merkmale nicht nur nicht wahrnehmbar: es ist für Beobachter nicht einmal erkennbar, dass es zu diesem Zeitpunkt etwas zu sehen gegeben hätte!

Weiters kann eine Testperson, ohne dass eine Interpretationsleistung höherer Ordnung, Bcgriffsbildung oder (verbale) Kommunikation nötig ist, rein motorisch reagieren, wobei bei einfachen Aufgabestellungen das Blicken, Fixieren, Verfolgen einer Bewegung und Suchbewegungen der Augen nach Entschwinden eines Objektes zur Verifikation genügen. Bei bestimmten Fragestellungen (z.B. Stereotests, Farbtests) ist eine zusätzliche Differenzierung nötig, die die Testperson ebenso bevorzugt motorisch erbringen können soll; durch Hinzeigen, Drücken von Tasten, Bewegen eines Joysticks, haptisches Ergreifen bereitliegender Gegenstände, Zeigen auf eine (weitere) Tafel, Antippen auf einem zweiten Monitor etc. Auf Grund der Einfachheit dieser geradezu intuitiven Bestätigung ergibt sich die oben angesprochene hervorragende Eignung für Sereening-Verfahren.

Um sowohl die Vorgangsweise beim Erheben des Sehvermögens zügig und effizient, als auch die Auswertung reproduzierbar und unabhängig von der subjektiven Interpretation eines Prüfers zu gestalten, ist es sinnvoll, die Darstellung der integralen und differentialen Objekte unter Einsatz geeigneter technischer Vorrichtungen mit der (motorischen) Reaktion der Testperson zu koppeln. Unter Berücksichtigung einer personenspezifischen Reaktionszeit erlaubt eine Zcitmessvorrichtung, insbesonders bei dynamischen oder mobiler Darstellung der Objekte, ob zentral, in einem Sehfeld, das der Größe der Makula entspricht, oder im peripheren Gesichtsfeld, die Zuordnung auf die Art und/oder den Ort der Präsentation, ab dem oder bis zu dem das integrale oder differentiale Objekt wahrgenommen wurde.

Wird die oben beschriebene Koppelung durchgeführt, können bei entsprechender Datenerfassung und Auswertung nicht nur die Grenzwerte des Sehvermögens am Beginn eines Sehtests, sondern auch die Veränderung der Qualität und der Reaktionszeit im Laufe des Tests erhoben und präzise dokumentiert werden. So können, alltägliche Sehsituationen repräsenticrend, anspruchsvolle und andauernde Sehaufgaben gestellt werden, durch die die Zuverlässigkeit und Belastbarkeit des visuellen Systems mit objektiven Messwerten verifiziert werden kann. Die Erhebung dieser Daten über einen längeren Zeitraum des Sehtests samt ihrer Auswertung ist so gestaltbar, dass sie eine hohe Signifikanz für subjektiv empfundene und bisher nicht objektivierbare Sehprobleme und -beschwerden im Alltag aufweisen.

Wenngleich damit die Erfindung nicht unmittelbar beschrieben wird, so ist doch illustrativ darauf zu verweisen, dass ihre Anwendung auch eine neue Art der Dokumentation erforderlich macht:

Zur Bestimmung der SEHSCHÄRFE (= Auflösungsvermögen) wird der kleinste Winkel ermittelt, unter dem die Richtung der Öffnung eines Landoldtringes bzw. die Art einer Optotype noch erkannt wird. Als Einheit wird bisher eine Größe mit der Bezeichnung "Visus = 1,0" verwendet. Damit ist definiert, dass die Öffnung des Landoldtringes dem Beobachterauge unter 1/60 Altgrad (= 1 Winkelminute) dargestellt wird, das ist auf 1 m Entfernung gerechnet ca. 0,29 mm. Vielfache oder Teile davon wurden früher (und bis heute im angelsächsischen Raum) mit Bruchzahlen gebildet; in letzter Zeit ist es üblich geworden, den so definierten Visus durch gerundete Dezimalzahlen nach einer geometrischen Reihe als Kehrwertfunktion des Auflösungsvermögens in Winkelminuten anzuschreiben (2,0 - 1,6 - 1,25 - 1,0 - 0,8 - 0,63 - 0,5 - 0,4 - 0,32 - 0,25 - 0,2 - 0,16 - 0,125 - 0,1 - 0,08 usw.).

Normgemäß erfolgt die Visusmessung mittels Optotypen und unter Beleuchtungsvorschriften, nach denen unter anderem die Testtafel mindestens 4 mal so hell (bis maximal 10 mal so hell) sein muss wie die Umgebung. Die vorliegende Erfindung unterscheidet sich grundsätzlich von dieser lichttechnischen Hervorhebung des Testfeldes, sodass zum Zwecke einer unmissverständlichen Bezeichnung die Verwendung einer anderen, zeitgemäßeren Winkeleinheit empfohlen wird.

Die SI-Einheit für Entfernung ist Meter. Bei integralen Objekten ist der Abstand der beiden Gegenpole zu beschreiben. Für die optometrische Praxis ist dazu nur die Angabe eines Winkels sinnvoll. Der Flächenwinkel ist eine Verhältniszahl aus Größe durch Entfernung, nach exakter Definition die Länge eines Kreisbogens durch zugehörigen Radius, mit der Bezeichnung "Radiant" (rad), Das ergibt für das minimum separabile zunächst sehr kleine Zahlen mit vielen Nachkomma-Nullen. Nimmt man, wie bei der Einheit dB für die Lautstärke, davon den dekadischen Logarithmus, multipliziert den Wert mit 10 und lässt das Minus weg, bekommt man zweistellige Zahlen. ( $y[dR] = -10 \cdot {}^{10}\log x[rad]$ und umgekehrt: $x[rad] = 10^{-y[dR]/10}$ ). Diese praxistaugliche, ebenso dimensionslose Winkeleinheit könnte etwa sinnvoll mit "deziRadiant", abgekürzt dR, bezeichnet werden.

Für die Anwendung in der Praxis ist ein erster Vorteil, dass Werte nach dieser Einheit nicht nur einfach mit Taschenrechner oder Tabelle, sondern nach kurzer Übung jederzeit im Kopf auf Größen in der Realität umgelegt werden können und umgekehrt. Zum Zweiten: verwendet man nur ganze Zahlen für dR entspricht der Faktor dieser geometrischen Reihe mit $\sqrt[10]{10}$ genau den schon recht gebräuchlichen, logarithmischen Visusstufen, allerdings ohne auf dem Altgrad bzw. der Winkelminute aufzubauen. Vorgeschlagen wird, Werte in deziRadiant zur Abgrenzung gegenüber bisherigen Messverfahren nicht nur wegen der anderen Darstellungsart, sondern auch aufgrund der neu definierten Leuchtdichtevorschrift nicht in Visus umzurechnen.

Ähnliches gilt für die Dokumentation der relativen Leuchtdichteunterschiede: der KONTRAST ist prinzipiell eine Verhältniszahl für zwei Flächen mit unterschiedlicher Leuchtdichte. Bei der bisherigen Berechnung nach Michelson (für periodische Muster in Verwendung) steht im Nenner $|L_{max} + L_{min}|$, in der Formel nach Weber (gängig angewandt für relativ kleine, dunkle Optotypen auf weißem Grund ) steht im Nenner nur $L_{max}$. Dies ergibt beim Menschen Werte, die von K = 1 bis ca. 0,005 gehen. Für integrale und differentiale visuelle Objekte sollte (bei niedrigen Kontrasten) im Nenner für $K_{integral}$ die Referenzleuchtdichte $L_{Tf}$ stehen. Aus dieser dimcnsionslosen Verhältniszahl x für $K_{integral}$ soll wie oben der dekadische Logarithmus gezogen werden, danach ist diese Zahl mit minus 10 zu multiplizieren: (y [dC] = -10 · $^{10}$log x[K] und umgekehrt: x[K] - $10^{-y[dC]/10}$). Als eindeutige Bezeichnung sei "deziContrast", abgekürzt dC, zur Abgrenzung von gängigen Formeln nach Michelson oder Weber vorgeschlagen. Wählt man ganze Zahlen für dC ergibt sich eine praktikable Stufung mit dem Faktor $^{10}\sqrt{10}$ und Werte bis ca. 23dC an der menschlichen Kontrostschwelle.

Für die Anwendung in der Praxis ist im Sinne der Erfindung gegenüber bisheriger optometrischer Gewohnheit immer die Leuchtdichte L [cd/m$^2$] zu beachten. Da weiters die Kontrastleistung [dC] der für das Sehvermögen im Alltag bedeutendere Wert ist, aber auch der Auflösungswinkel [dR] Beachtung finden soll und beide einander beeinflussen, wird folgende Schreibweise vorgeschlagen: dCR(L). Der erste Wert ist $K_{integral}$ in deziContrast nach obiger Definition, der zweite Wert der Auflösungswinkel in dR nach gleicher mathematischer Vorschrift vom Radiant hergeleitet, und L die Leuchtdichte in cd/m$^2$.

## 4. Beschreibung der Abbildungen:

**Fig. 1:** Stellt das Prinzip der visuellen Objekte im Sinne der Erfindung dar: die durchschnittliche Leuchtdichte der Flächen eines Objektes (L) ist gleich der Leuchtdichte des Testfeldes ($L_{Tf}$), dass also gilt:
($A_1 · L_1$) + ($A_2 · L_2$) = ($A_1$ + A2)· $L_{Tf}$, wobei $A_1$ und $A_2$ die Flächen des Objektes sind.

**Fig. 2:** Stellt ein integrales Objekt dar. Sie zeigt im Unterschied zur Figur 1, dass die Übergänge an der Grenze zwischen den Flächen nicht nur abrupt, sondern auch beliebig fließend gestaltet werden können, hier mit linearen Steigungen unterschiedlicher Größe.

**Fig. 3:** Zeigt in einem Diagramm den Leuchtdichteverlauf an einer Objektkante, wobei die x-Achse den Ort im Prinzip eines Schnittes, die y-Achse die Leuchtdichte an dem jeweiligen Ort darstellt. Die strichlierte Linie zeigt die scharfe Kante in der Realität (links dunkler, rechts heller), die durchgezogene Linie beispielhaft den Leuchtdichteverlauf auf der Netzhaut nach der Abbildung durch das Auge.

**Fig. 4:** Zeigt schematisch die erste Ableitung der Funktion (aus der durchgezogenen Linie der Figur 3). Die maximale Steigung und damit die stärksten Änderung im Leuchtdichteverlauf bildet nun den oberen Umkehrpunkt. In der Realität entspricht die x-Achse dieses Diagramms der Leuchtdichte eines Testfeldes, der Graph stellt eine helle Linie oder Optotype darauf dar.

**Fig. 5:** Zeigt die zweite Ableitung der Funktion aus Fig. 3, deren maximale Leuchtdichtenänderung hier nun dem Nulldurchgang entspricht. Weiters sind die Flächen ober- und unterhalb der der x-Achse (Leuchtdichte des Testfeldes) gleich groß. Diesem Prinzip entsprechen die integralen Objekte im Sinne der Erfindung zur Darstellung beliebiger Kontraste.

**Fig. 6:** Zeigt die dritte Ableitung der Funktion aus Fig. 3. Die erste Potentialänderung erhält nun, nachdem sie ins Gegenteil umgeschlagen ist, einen Gegenpol. Die Flächen zwischen Graph und x-Achse heben einander auf, damit ist die durchschittliche Leuchtdichte des Objektes und jene des Testfeldes ident. Der Abstand der beiden Nulldurchgänge ist das Abstandskriterium. In der Realität entsprechen diesem Diagramm die differentialen Objekte im Sinne der Erfindung, womit zusätzlich zum Kontrast auch ein beliebiger Auflösungswinkel darstellbar ist.

**Fig. 7:** Zeigt eine erste, einfache Darstellung eines differentialen Objektes.

**Fig. 8:** Zeigt wie Fig. 7 ein differentiales Objekt, allerdings mit umgekehrtem ("negativem") Leuchtdichteverlauf.

**Fig. 9:** Zeigt eine Möglichkeit, wie mit Hilfe einer differentialen Linie eine Erhebung des Schvermögens durchgeführt werden kann. Beobachter müssen die Uhrzeit angeben oder zeigen, wo der Zeiger steht.

**Fig. 10:** Zeigt ein mittels integraler Linie gebildetes Piktogramm. Die Linienbreite, Kantengestaltung, Kontrast, Dynamik der Erscheinung, etc. kann unabhängig von der Gesamtgröße des Objektes variieren.

**Fig. 11:** Zeigt ein kurzes Wort, mittels integraler Linie dargestellt. Auch hier ist wie in Figur 10 die Darstellungsart der integralen Linie von der Wortgröße unabhängig.

**Fig. 12:** Zeigt verschiedene einfache Ausformungen des Differential-Ringes. Dieser kann in Größe, Proportion, Kantengestaltung, Erscheinungsform, Dynamik der Darstellung etc. beliebig variiert werden.

**Fig. 13:** Zeigt eine mögliche Form für ein sehr kleines differentiales Objekt, womit verschiedene geometrische Figuren (auch Buchstaben, Ziffern etc.) dargestellt werden können.

**Fig. 14:** Zeigt ein differentiales Objekt in anderer Leuchtdichtenabfolge wie Fig. 13, die innere Form ist ungleich der äußeren. Damit sind in den Flächen des Objektes andere Farben verwendbar als auf dem Testfeld. Probanden erkennen das ganze Objekt aufgrund des Farbunterschicdes zum Testfeld, Aufgabe ist aber, die eingeschriebende Form im Objekt zu differenzieren und anzugeben.

**Patentansprüche**

1. Visuelle Objekte, dargestellt mit gängigen Mitteln einschließlich Projektion auf die Netzhaut etwa per Refraktion oder Interferenz zur Optimierung (subjektive Brillengtasbestimmung) und Erhebung des Sehvermögens, insbesondere Grenzwerte für Sehschärfe, Kontrastleistung, Binokular- und Stereosehen, Belastbarkeit, Adaptionsfähigkeit und -geschwindigkeit, Gesichtsfeldgröße für hohe und niedrige Kontraste, **gekennzeichnet dadurch, dass** die durchschnittliche Leuchtdichte des singulären visuellen Objektes gleich ist der Leuchtdichte des Hintergrundes oder Testfeldes, auf dem das Objekt dargestellt wird, jedenfalls aber die Differenz der Leuchtdichten der dem Objekt zugehörigen Flächen höher ist als die Differenz der Leuchtdichte irgendeiner Fläche des Objektes zu der Leuchtdichte des Testfeldes oder Hintergrundes.

2. Objekte nach Anspruch 1, **dadurch gekennzeichnet, dass** die Leuchtdichtenänderung an der Grenze zwischen den Flächen abrupt oder verlaufend erfolgt.

3. Objekte nach Anspruch 1 und 2, **dadurch gekennzeichnet, dass** die Leuchtdichte des Testfeldes gleich ist der Gcsichtsfeldleuchtdichte mit möglichst geringen Abweichtutgen, wobei diese Leuchtdichte in verschiedenen Intensitäten für photopisches bis skotopisches Sehen hergestellt wird.

4. Visuelle Objekte nach den Ansprüchen 1 bis 3, **gekennzeichnet dadurch, dass** sogenannte integrale Objekte Verwendung finden, die mathematisch der zweiten Ableitung einer Objektkante entsprechen und deren Kennzeichen die fotometrische Symmetrie ist, indem die beiden einander entsprechenden Flächen im Mittelwert die Leuchtdichte des Hintergrundes aufweisen.

5. Visuelle Objekte nach den Ansprüchen 1 bis 3, **gekennzeichnet dadurch, dass** sogenannte differentiale Objekte Verwendung finden, die mathematisch der dritten Ableitung einer Objektkante entsprechen und deren Kennzeichen die fotometrische Polarität ist, indem die beiden in ihrer Leuchtdichte ähnlichen äußeren Flächen gemeinsam mit der Leuchtdichte der umschlossenen Fläche die durchschnittliche Leuchtdichte des Hintergrundes ergeben, sodass für diese Objekte - zusätzlich zum Kontrast - der Abstand der Gegenpole (als Auflösungswinkel) festzulegen ist und variieren kann.

6. Visuelle Objekte nach den Ansprüchen 1 bis 5, **gekennzeichnet dadurch, dass** die Leuchtdichte ihrer Teilflächen von dunkel/ hell auf hell/dunkel umgekehrt wird (positiv/negativ).

7. Visuelle Objekte nach den Ansprüchen 1 bis 6, **dadurch gekennzeichnet, dass** die Erscheinungsart der Objekte bei Verwendung geeigneter Darstellungsmittel dynamisch und variabel sein kann, sodass ein Einschleifen des Erscheinens ebenso möglich ist wie ein Oszillieren, etwa zwischen positiver und negativer Darstellung, oder ein abruptes Erscheinen und umgekehrt ebenso variables Entschwinden.

8. Visuelle Objekte nach den Ansprüchen 1 bis 7, **dadurch gekennzeichnet, dass** die Objekte als Figuren dargestellt werden, was von der Darstellung einer einzelnen integralen oder differentialen Linie, daraus gebildeter Figuren wie Piktogramme, Wörter oder ähnlichem bis zu derart kleinen Figuren reicht, dass innerhalb der Außengrenzen des Objektes keine Leuchtdichte vorhanden ist, wie sie der Testfläche als Hintergrund entspricht.

9. Visuelle Objekte nach den Ansprüchen 1 bis 8, **dadurch gekennzeichnet, dass** mehrere Objekte gleichzeitig dargestellt werden, unabhängig voneinander erscheinen oder entschwinden oder eines oder mehrere Objekte bewegt werden, eine Bewegung in sich darstellen, den Ort am Testfeld oder die Position gegenüber anderen Objekten verändern.

10. Visuelle Objekte nach den Ansprüchen 1 bis 9, **dadurch gekennzeichnet, dass** nach gängigen Trennverfahren bzw. durch unterschiedliche Netzhautprojektionen dem rechten und linken Auge gleichzeitig unterschiedliche Objekte oder gleiche, aber in ihrer horizontalen Position minimal verschobene Objekte, präsentiert werden.

11. Visuelle Objekte nach den Ansprüchen 1 bis 10, **dadurch gekennzeichnet, dass** in den Flächen der Objekte ebenso wie auf dem Testfeld verschiedene Farbtemperaturen, Farbbänder oder Spektralfarben Verwendung finden, wobei die Teilflächen eines Objektes bevorzugt unterschiedliche Leuchtdichten bei sonst gleichen Farbanteilen oder Wellenlängen aufweisen.

**12.** Visuelle Objekte nach den Ansprüchen 1 und 2, 4 bis 11, **dadurch gekennzeichnet, dass** für das Gesichtsfeld, das das Testfeld umgibt insgesamt oder an definierten Flächen, auf Dauer oder für eine kurze Zeit eine höhere Leuchtdichte hergestellt wird als jene des Testfeldes (= Blendung durch das Umfeld), als auch umgekehrt für das Testfeld eine beabsichtigte höhere Leuchtdichte gegenüber dem Umfeld (= Blendung durch das Testfeld).

**13.** Methode zur Auswertung der Reaktion einer Testperson auf Objekte nach den Ansprüchen 1 bis 12, **gekennzeichnet dadurch, dass** Testpersonen den Beginn bzw. das Ende einer visuellen Wahrnehmung mittels einer motorischen Aktion wie Demunstration, Antippen von Darstellungen auf einer (weiteren) Tafel, Vorrichtung oder Mönitor, haptisches Ergreifen entsprechender Gegenstände und Ähnliches, bevorzugt aber einfach durch Augenbewegungen wie Blicken, Fixieren, Verfolgen eines mobilen Objektes und Suchbewegungcn bei Verschwinden eines sich verändernden Objektes, bestätigen.

**14.** Methode zur Auswertung der Reaktion einer Testperson auf Objekte nach den Ansprüchen 1 bis 13, **dadurch gekennzeichnet, dass** durch Koppelung der Darstellung der Objekte mit der (motorischen) Reaktion bei Beobachtern in Kombination mit einer Zeitmessvorrichtung die Zeitdauer sowie ihre Veränderung während eines subjektiven Messvorganges gemessen und gespeichert wird, sowie bei sich verändernden Darstellungsformen eine Zuordnung zwischen der Art und/oder dem Ort der Objekte einerseits und des Zeitpunktes der Wahrnehmung bzw. des Verschwindens andererseits herstellbar ist.

**Claims**

**1.** Visual objects, presented with common means, including projection to the retina by refraction or interference for optimisation (subjective ophthalmic lens refraction) and determination of vision, in particular the thresholds for acuity, contrast, binocular and stereo vision, resilience, adaptation capacity and speed, size of field of vision for high and low contrast, **characterised by** the average luminance of the singular visual object being equal to the luminance of the background or test field on which the object is presented, but with the difference between luminances of the areas allocated to the object being higher than the difference of the luminance of any area of the object to the luminance of the test field or background.

**2.** Objects according to claim 1, **characterised by** the luminance change at the threshold between the areas being abrupt or gradual.

**3.** Objects according to claims 1 and 2, **characterised by** the luminance of the test field being equal to the field of vision luminance with the lowest possible deviations, with this luminance being created in different intensities for photopic to scotopic vision.

**4.** Visual objects according to claims 1 to 3, **characterised by** so-called integral objects being used that mathematically correspond to the second derivation of an object edge, and that are marked by the photometric symmetry by both corresponding areas having the background luminance on average.

**5.** Visual objects according to claims 1 to 3, **characterised by** so-called differential objects being used that mathematically correspond to the third derivation of an object edge and that are marked by photometric polarity by the two outer areas similar in their luminance and the luminance of the surrounded area resulting in the average background luminance, so that the distance of the counterpoles (as the resolution angle) can be determined and varied for these objects - in addition to contrast.

**6.** Visual objects according to claims 1 to 5, **characterised by** the luminance of their partial areas being inverted from dark/light to light/dark (positive/negative).

**7.** Visual objects according to claims 1 to 6, **characterised by** the appearance type of the objects when using suitable presentation means being dynamic and variable, so that looping in of the appearance being possible as well as oscillation, e.g. between positive and negative presentation, or abrupt appearance and, on the other hand, also variable disappearance.

**8.** Visual objects according to claims 1 to 7, **characterised by** the objects being presented as figures, which ranges from the presentation of individual integral or differential lines, figures formed from them like pictograms, words or similar, down to figures so small that there is no luminance present within the object's outer borders that corresponds to the test area as background.

**9.** Visual objects according to claims 1 to 8, **characterised by** several objects being presented at the same time, appear or disappear independently of each other or that one or several objects are moved, form a movement in themselves, change the location on the test field or change the position in relation to the other objects.

10. Visual objects according to claims 1 to 9, **characterised by** common separating procedures or different retina projection on the right and left eyes being used to present different objects or same objects with small deviations in their horizontal position at the same time.

11. Visual objects according to claims 1 to 10, **characterised by** different colour temperatures, colour bands or spectral colours being used in the areas of the objects, with the partial areas of an object preferably comprising different luminances at otherwise equal colour shares or wavelengths.

12. Visual objects according to claims 1 and 2, 4 to 11, **characterised by** assembling a higher luminance of the ambient field of vision, overall or on defined areas, compared to the test field within (=glare of the field of vision), this being permanently or for certain periods of time, as well as vice versa a deliberately higher luminance of the test field compared to the field of vision (=glare of the test field).

13. Method for assessment of the test person's reaction to objects according to claims 1 to 12, **characterised by** test persons confirming the start or end of any visual perception by a motor action like demonstration, tapping of presentations on (another) panel, device or screen, haptic taking up of the corresponding objects and similar, but preferably by simple eye movement like looking, fixating, following a mobile object and search movements when a changing object disappears.

14. Method for assessment of the test person's reaction to objects according to claims 1 to 13, **characterised by** linking the presentation of objects to the observers (motor) reaction, in combination with a time measurement device, that is used to record the duration and the changes during the subjective measurement process, and to permit allocation between the type and/or location of the objects on one hand and the time of perception or disappearance on the other hand for varying presentation types.

**Revendications**

1. Objets visuels représentés de manière y.c. par la projection sur la rétine, par réfraction ou par interférence, pour optimisation (détermination subjective d'un verre correcteur) et évaluation de l'aptitude visuelle, en particulier des valeurs limites de l'acuité visuelle, perception des contrastes, vision binoculaire et stéréoscopique, point de saturation, capacité et vitesse d'adaptation, le champ visuel par fort et faible contraste, **caractérisés par le fait que** la luminance moyenne de l'objet visuel singulier est la même que celle de l'arrière-plan ou de l'espace visuel sur lequel l'objet est présenté, mais dans tous les cas, que la différence entre les luminances des surfaces appartenant à l'objet est supérieure à la différence entre la luminance de n'importe quelle surface de l'objet et l'espace visuel ou l'arrière-plan.

2. Objets comme dans la revendication 1, **caractérisés par le fait que** la variation de luminance à la limite des surfaces s'effectue de manière abrupte ou douce.

3. Objets comme dans les revendications 1 et 2, **caractérisés par le fait que** la luminance de l'espace visuel est la même que celle du champ visuel avec des écarts aussi faibles que possible, cette luminance étant générée à des intensité différentes pour passer d'une vision photopique à une vision scotopique.

4. Objets visuels comme dans les revendications 1 à 3, **caractérisés par** l'utilisation d'objets issus du calcul intégral correspondant mathématiquement à la dérivée seconde de l'arête d'un objet et dont le signe distinctif est la photométrie symétrique, de manière que les deux surfaces qui se correspondent présentent en moyenne la même luminance que l'arrière-plan.

5. Objets visuels comme dans les revendications 1 à 3, **caractérisés par** l'utilisation d' objets issus du calcul différentiel correspondant mathématiquement à la dérivée troisième de l'arête d'un objet et dont le signe distinctif est la polarité photométrique, des deux surfaces extérieures d' une luminance semblable et de la luminance de la surface entourée il résulte la luminance moyenne de l'arrière-plan, si bien qu'il convient pour ces objets - en plus du contraste- de définir la distance des contrepôles (comme angle de discrimination), celle-ci pouvant varier.

6. Objets visuels comme dans les revendications 1 à 5, **caractérisés par le fait que** la luminance de leurs faces partielles est inversée, obscur/clair devient clair/obscur (positif/négatif).

7. Objets visuels comme dans les revendications 1 à 6, **caractérisés par le fait que** les objets, moyennant l'emploi de moyens de présentation adéquats, peuvent paraître dynamiques et variables, de sorte que l'on peut obtenir une apparition progressive aussi bien qu'oscillante, par exemple entre une représentation positive et négative, ou une apparition soudaine tout comme une disparition variable ou vice versa.

8. Objets visuels comme dans les revendications 1 à 7, **caractérisés par le fait que** les objets sont représentés sous forme de figures, ce qui peut aller de la représentation d'une ligne intégrale ou différentielle unique, à des figures du genre pictogrammes constitués de ces lignes, des mots et autres, jusqu'à des figures si petites, qu'il n'existe pas, au dedans des limites extérieures de l'objet, de luminance correspondant à celle de la surface d'essai en arrière-plan.

9. Objets visuels comme dans les revendications 1 à 8, **caractérisés par le fait que** plusieurs objets sont représentés simultanément, apparaissent ou disparaissent indépendamment les uns des autres, ou un ou plusieurs objets sont déplacés, représentent un mouvement en eux-mêmes, changent de place dans l'espace visuel ou par rapport à d'autres objets.

10. Objets visuels comme dans les revendications 1 à 9, **caractérisés par le fait que**, par des méthodes de séparation courantes, ou bien par des projections différentes sur la rétine, on présente à l'oeil droit ou gauche simultanément des objets différents ou bien semblables mais très légèrement décalés par rapport à leur position horizontale.

11. Objets visuels comme dans les revendications 1 à 10, **caractérisés par** l'utilisation dans les surfaces des objets ainsi que dans l'espace visuel, de températures de couleur, bandes de couleur ou spectres de couleur différents, les faces partielles d'un objet présentant de préférence des luminances différentes, les composantes de couleur et les longueurs d'onde restant cependant identiques.

12. Objets visuels comme dans les revendications 1 et 2, 4 à 11, **caractérisés par le fait qu'**est générée dans le champ visuel qui cerne l'espace visuel, dans l'ensemble ou sur des surfaces définies, durablement ou temporairement, une luminance plus forte que celle de l'espace visuel (= éblouissement par l'environnement), ou en revanche une luminance intentionnellement plus forte dans l'espace visuel que dans le champ visuel (= éblouissement par l'espace visuel).

13. Méthode d'interprétation de la réaction d'un sujet soumis aux tests réalisés à l'aide des objets 1 à 12 **caractérisée par le fait que** le sujet approuve le début ou la fin d'une perception visuelle au moyen d'une action motrice telle qu'une démonstration, le toucher d'une figure sur un (autre) tableau, mécanisme ou écran, la saisie haptique d'objets correspondants ou autres, mais de préférence par des mouvements oculaires comme regarder, fixer, suivre un objet mobile, et des mouvements de recherche lors de la disparition d'un objet en évolution.

14. Méthode d'interprétation de la réaction d'un sujet soumis aux tests réalisés à l'aide des objets 1 à 13 **caractérisée par le fait que** l'association de la représentation des objets avec la réaction (motrice) des observateurs jointe à un dispositif de comptage horaire permet la mesure et l'enregistrement de la durée ainsi que sa fluctuation lors d'une évaluation subjective, dans le cas des représentations en évolution il est aussi possible d'établir un lien entre la nature et/ou le lieu des objets d'une part et le moment de leur perception ou, le cas échéant, de leur disparition d'autre part.

Fig. 1

Fig. 2

Fig. 3

Fig. 4

Fig. 5

Fig. 6

Fig. 7

Fig. 8

**Fig. 9**

**Fig. 10**

**Fig. 11**

**Fig. 12**

**Fig. 13**

**Fig. 14**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2247087 A
- US 20060152675 A